# EUROPEAN PATENT SPECIFICATION

(11) **EP 2 114 407 B1**
(45) Date of publication and mention of the grant of the patent: **06.04.2011**
(21) Application number: 08700996.5
(22) Date of filing: 07.01.2008
(51) Int. Cl.: A61K 31/4965, A61P 11/00

(54) **USE OF SODIUM BLOCKERS FOR AN EARLY THERAPY OF OBSTRUCTIVE LUNG DISEASES**
VERWENDUNG VON NATRIUMBLOCKERN ZUR FRÜHZEITIGEN BEHANDLUNG VON OBSTRUKTIVEN LUNGENERKRANKUNGEN
UTILISATION D'INHIBITEURS SODIQUES POUR LE TRAITEMENT PRÉCOCE DE MALADIES PULMONAIRES OBSTRUCTIVES

(30) Priority: 08.01.2007 EP 07000267
(43) Date of publication of application: 11.11.2009
(73) Proprietor: Universität Heidelberg, 69117 Heidelberg (DE)
(72) Inventor: MALL, Marcus, 69117 Heidelberg (DE)
(74) Representative: Müller-Boré & Partner Patentanwälte
(86) International application number: PCT/EP2008/000051
(87) International publication number: WO 2008/083942

(56) References cited:
- WO-A-2006/023573
- US-B1- 6 323 187
- MALL M ET AL: "Increased airway epithelial Na+ absorption produces cystic fibrosis-like lung disease in mice" NATURE MEDICINE, NATURE PUBLISHING GROUP, NEW YORK, NY, US, vol. 10, no. 5, May 2004 (2004-05), pages 487-493, XP002313818 ISSN: 1078-8956
- MOCHIZUKI H ET AL: "Effect of inhaled amiloride on water-induced bronchoconstriction in asthmatic children." AMERICAN JOURNAL OF RESPIRATORY AND CRITICAL CARE MEDICINE AUG 1994, vol. 150, no. 2, August 1994 (1994-08), pages 555-557, XP009085086 ISSN: 1073-449X
- HIRSH ANDREW J ET AL: "Evaluation of second generation amiloride analogs as therapy for cystic fibrosis lung disease" JOURNAL OF PHARMACOLOGY AND EXPERIMENTAL THERAPEUTICS, vol. 311, no. 3, December 2004 (2004-12), pages 929-938, XP002437463 ISSN: 0022-3565
- YERXA B R ET AL: "P2Y2 receptor agonists: Structure, activity and therapeutic utility" DRUGS OF THE FUTURE, BARCELONA, ES, vol. 24, no. 7, July 1999 (1999-07), pages 759-769, XP002387694 ISSN: 0377-8282
- TIERNEY L; MCPHEE S; PAPADAKIS M: "Current Medical Diagnosis & Treatment 2000" 2000, MCGRAW-HILL , NEW YORK * page 284 - page 289 *

## Description

The present invention relates to a blocker of sodium channels in cell membranes, particularly in membranes of epithelial cells of organs belonging to the respiratory tract to be used as the pharmaceutically active ingredient in a medicament for treating an obstructive lung disease in a patient.

Obstructive lung diseases like cystic fibrosis (CF), neonatal chronic lung disease (CLD), also known as bronchopulmonary dysplasia (BPD), asthma bronchiale and chronic bronchitis (also known as chronic obstructive pulmonar disease; COPD) belong to the most common chronic diseases in Western Europe and North America. While CF is the most common fatal hereditary disease in the white population, CLD is a frequent health problem of premature infants. Asthma bronchiale is one of the most common chronic diseases of children and asults. Cigarette smoke induced COPD is currently the fourth leading cause of death worlwide. All chronic obstructive lung diseases are accompanied by various degrees of mucus obstructions, goblet cell metaplasia and chronic inflammation of the respiratory tract and the formation of emphysemas, i.e. disturbance in development or a destruction of alveoli, resulting in a respiratory insufficiency. To this date only limited therapies, which are primarily oriented on the symptoms, like e.g. administration of β-mimetics, corticosteroids, anticholinergics, antibiotics, and mucolytics to a patient and physiotherapy are available for the therapy of these diseases. Therefore, a new effective therapy of obstructive lung diseases is of high clinical and socioeconomic interest.

In the respiratory tract of CF patients there is a defect in the cAMP-dependent secretion of chloride and an enhancement in the resorption of sodium (Knowles M.R. et al., Science 1983;221:1067-1070). These characteristic defects of the epithelial transport of ions leads primarily to a dehydration (depletion of volume) of the surface of the respiratory tract and therefore to a defect of the mucociliar clearance and the pulmonal defense. In the case of asthma, CLD, COPD, and most of the other obstructive lung diseases, there is primarily an inflammation of the respiratory tract with mucus hypersecretion which results in a secondary dehydration of the surface of the respiratory tract and therefore also results in a defect of the mucociliary clearance.

The importance of the absorption of sodium in the *in vivo* pathogenesis of chronic obstructive lung diseases has also been shown in transgenic mouse models (Mall M et al., Nat Med 2004;10:487-493). It should be noted that over-expression of the β-subunit of the epithelial sodium channel (ENaC, also known as SCNN1) in the respiratory tract of mice also results in spontaneous lung diseases having a great similarity to CF as well as to other chronic obstructive lung diseases of human beings (CF, CLD, asthma bronchiale, chronic bronchitis, COPD). This means that the respiratory tract of β-ENaC overexpressing mice is dehydrated on the surface which leads to a defect of the mucociliary clearance, mucus obstruction, goblet cell metaplasia, chronic inflammation, and the formation of emphysema.

Further, the above observation, i.e. that a dehydration of the surface of the respiratory tract of CF patients leads to a chronic obstructive lung disease, forms the rationale for a therapy with specific inhibitors of the epithelial sodium channels. This strategy should inhibit the resorption of liquids by the surface of the respiratory tract so that the hydration of the surface film and the mucociliar clearance is improved and therefore antagonizes the mucus obstruction. Recent tests for the therapeutic effectiveness of an aerosol of the classic sodium channel blocker amiloride for the treatment of the lung disease of CF-patients, however, did not show any therapeutic effect (Graham A. et al., Eur Respir J 1993;6:1243-1248; Bowler IM et al., Arch Dis Child 1995;73:427-430; Pons G. et al., Pediatr Pulmonol 2000;30:25-31).

Hochizuki H. et al. (American Journal of Respiratory and Critical Core Medicine 1994: Vol. 150, No. 2, 555-557) describe the effects of amiloride on bronchoconstriction induced by ultrasonically nebulized destilled water in asthmatic children. Hirsh AJ et al. (Journal of Pharmacology and Experimental Therapeutics 2004: Vol. 331, No. 3, 929-938) describe second generation amiloride analogs and compare these to amiloride.

So far the aerosol therapy with the sodium channel blocker amiloride has been exclusively carried out for CF patients having an advanced lung disease, wherein the minimum age of the patients in said studies was five years. In this group of patients the treatment with amiloride had no therapeutic success.

Thus, the problem underlying the present invention is to provide a new method for a successful *in vivo* therapy of obstructive lung diseases using sodium channel blockers.

The solution to the above technical problem is achieved by the embodiments characterized in the claims.

In particular, the present invention relates to the use of at least one sodium channel blocker as a pharmaceutically active ingredient of a medicament for treating an obstructive lung disease in a susceptible patient prior to having substantial mucus obstruction or secondary, disease related changes of the lung. Therefore, in one embodiment the present invention relates to a sodium channel blocker for use in the prevention or early therapy of an obstructive lung disease in a patient, wherein the early therapy is carried out in an early phase of the disease characterized by the lack of any substantial mucus obstruction or secondary, disease related changes of the lung.

The sodium channel to be blocked may be any sodium channel in cell membranes, particularly in membranes of epithelial cells of organs belonging to the respiratory tract. The organ of the respiratory tract may be for example the trachea or the lung including bronchi, bronchioles, and alveoli. In a preferred embodiment of the present invention, the sodium channels to be blocked are situated in membranes of epithelial cells of the lung.

The term "medicament" as used herein relates to any pharmaceutical composition comprising at least one sodium channel blocker in a pharmaceutically effective amount.

According to the present invention, the medicament may be administered by any administration route known in the art being suitable for delivering a medicament to the epithelium of an organ belonging to the respiratory tract. The route of administration does not exhibit particular limitations and includes for example inhalation, e.g. intrapulmonal, or nasal administration, systemic administration, e.g. by oral or intravenous route, and topic application, e.g. as an ointment. The medicament may be administered in any form known in the art, e.g. as a liquid, a powder, an aerosol, a capsule, or a tablet.

In a preferred embodiment of the present invention, the medicament is administered by an intrapulmonal application as an aerosol. The medicament according to the present invention may be for example inhaled, e.g. by using special devices or nebulizers, which administer the medicament in a fine spray that the patient breathes in. Further, suitable inhalers, which may be used for administering the medicament, like e.g. a metered-dose inhaler (MDI), which allows precise doses to be delivered directly to the lungs, are known to those skilled in the art. Such inhalers may use for example ozone-depleting chlorofluorocarbons or hydrofluoroalkane as propellants, but alternative delivery methods and propellants useful for delivering the medicament, like e.g. dry powder inhalers (DPIs), may also be used.

The term "early therapy" as used herein relates to a therapy that is initiated in a susceptible individual prior to the development of a lung disease (i.e. preventive) or in an early stage of an obstructive lung disease. This may be a preventive treatment that is initiated in a susceptible individual before the onset of a lung disease or a therapy for the treatment of an early stage of an obstructive lung disease, characterized e.g. by no progressed lung disease like mucus obstruction and no secondary changes of the lung, like e.g. airway remodelling, goblet cell metaplasia, chronic inflammation of the respiratory tract or emphysema, which may be evidenced e.g. by standard diagnostic tests including pulmonary function testing, pulmonary imaging, bronchoscopy with bronchoalveolar lavage.

Patients who are known to be susceptible to develop a chronic obstructive lung disease and will therefore benefit from a preventive or early therapy can be identified depending on the disease etiology as follows:
CF is an inherited multiorgan disease caused by mutations in the CFTR gene.

Lungs are normal at birth and the disease typically presents with gastrointestinal symptoms like meconium ileus, malabsorption and maldigestion due to pancreatic insufficiency, and failure to growth, i.e. the clinical diagnosis can often be established and confirmed by standard laboratory tests like sweat test or genetic testing in infancy prior to the onset of lung disease. Some patients are already identified before birth by prenatal screening for CFTR mutations. Further, neonatal CF screening programmes are currently being established in many countries that will allow to identify CF patients in the first weeks of life. Taken together, the majority of CF patients in the Western world are identified before the onset of chronic lung disease.

Neonatal CLD is caused by premature birth, i.e. patients at risk are readily identified and treatment can be commenced right after birth, i.e. before lung disease has developed.

Asthma is an episodic, recurrent disease characterized by reversible airway obstruction caused by various triggers including viral infections, allergens, physical exercise, or cold air. Typically, acute and recurrent episodes with reversible airflow obstruction due to mucus obstruction, goblet cell metaplasia, airway inflammation and related smooth muscle contraction alternate with symptom free episodes with no mucus obstruction, goblet cell metaplasia or inflammation in the absence of the trigger. Accordingly, "preventive or early therapy" could be commenced in a symptom free interval and preferably prevent or ameliorate the next asthma attack.

COPD typically starts in adulthood and is caused by chronic inhalation of cigarette smoke or other noxious particulates and/or toxicants. Since the establishment of chronic obstructive lung disease including mucus obstruction, inflammation, goblet cell metaplasia and emphysema takes several years, individuals at risk can be readily identified prior to the onset of lung disease, and the treatment commenced as a "preventive or early therapy" in individuals who smoke cigarettes or are exposed to occupational particulates and/or toxicants.

As used herein, a sodium channel blocker may be any molecule that is able to substantially decrease the ability of a sodium channel to transport sodium ions from the extracellular side of a cell membrane into the intracellular side of a cell membrane. In a preferred embodiment of the present invention, the sodium channel blocker is selected from the group consisting of amiloride, P2Y2-receptor agonists such as nucleotides, like e.g. ATP or UTP, or long-acting synthetic compounds like nucleotide analogs (e.g. Denufosol), and protease inhibitors, including e.g. aprotinin or BAY 39-9437 (a recombinant Kunitz-type serine protease inhibitor). In a particularly preferred embodiment of the present invention, the sodium channel blocker is amiloride (3,5-Diamino-N-(aminoiminomethyl)-6-chloro-pyrazinecarboxamide).

According to the present invention, the sodium channel blocker is used in a pharmaceutically effective amount. In a preferred embodiment of the present invention the sodium channel blocker is used in a amount ranging from about 0.1 mg/kg body weight to about 10 mg/kg body weight. In a more preferred embodiment of the present invention, amiloride is used in a amount ranging from about 0.3 mg/kg body weight to about 1 mg/kg body weight. In another more preferred embodiment of the present invention a P2Y2-receptor agonist is used in a amount ranging from about 1 mg/kg body weight to about 2 mg/kg body weight. In a further more preferred embodiment of the present invention a protease inhibitor is used in a amount ranging from about 0.3 mg/kg body weight to about 1 mg/kg body weight.

The term "obstructive lung disease" as used herein relates to a disease characterized by airflow limitation in the lung that develops over time. The obstructive lung disease according to the present invention may be associated with breathing-related symptoms, like e.g. cough, spitting or coughing mucus (expectoration), breathlessness upon exertion, progressive reduction in the ability to exhale, progressive shortness of breath, frequently accompanied by a phlegm-producing cough, with episodes of wheezing, irritation of the nose and throat, chest tightness or pain or a nonproductive cough. The above symptoms may vary, however, others may be present.

Examples of the obstructive lung disease according to the present invention are acute bronchitis which is usually caused by a virus and in most cases is self-limiting but can later develop either chronic bronchitis or asthma, and asthma which is characterized by attacks of coughing, wheezing, and shortness of breath (dyspnea).

In a preferred embodiment of the present invention the obstructive lung disease is a chronic obstructive lung disease. An example of such a chronic obstructive lung disease is chronic bronchitis (COPD) being characterized by chronic cough and sputum production, intermittent wheezing with variable degrees of shortness of breath on exertion. Other examples of chronic obstructive lung diseases are cystic fibrosis (CF) characterized by an increased transport of sodium across the respiratory tract lining which results in the dehydration of the liquid that lines the respiratory tract surface and neonatal chronic lung disease (CLD).

In another preferred embodiment of the present invention the obstructive lung disease is selected from the group consisting of cystic fibrosis (CF), neonatal chronic lung disease (CLD) and asthma bronchiale. In a more preferred embodiment of the present invention the obstructive lung disease is CF.

Further, the term "treatment" as used herein relates to the prevention and/or eradication or amelioration of disease related symptoms and/or disease related disorders. Obstructive lung diseases are often accompanied by pulmonal mortality, chronic inflammation of the respiratory tract, like e.g. pulmonal inflammation, mucus obstruction, resulting from secreted mucus, a viscous fluid composed primarily of highly glycosylated proteins called mucius suspended in a solution of electrolytes. Other disorders associated with obstructive lung diseases are goblet cell hyperplasia, goblet cell metaplasia being an important morphological feature in the respiratory tract of patients with chronic respiratory tract diseases, and emphysema which is a progressive destructive lung disease in which the walls between the alveoli in the lungs are damaged. Therefore, a preferred embodiment of the present invention is a use of at least one sodium channel blocker in the manufacture of a medicament for an early therapy of an obstructive lung disease as described above, wherein at least one disorder selected from the group consisting of pulmonal mortality, pulmonal inflammation, mucus obstruction, goblet cell metaplasia, cellular necrosis of epithelial cells, and emphysema is reduced in the patient, e.g. when compared to patients not being treated with a sodium channel blocker according to the present invention.

The reduction of the above symptoms and disorders as well as the success of an early therapy of an obstructive lung disease in a patient by use of a sodium channel blocker as described above can be monitored using methods known in the art. Examples of such methods for determining the presence and the course of the response to treatment of obstructive lung diseases are pulmonary function tests, like e.g. spirometry employing a spirometer, an instrument that measures the air taken into and exhaled from the lungs, or the testing of arterial blood gas by determining the amount of oxygen and carbon dioxide in the blood, wherein low oxygen (hypoxia) and high carbon dioxide (hypercapnia) levels are often indicative of chronic bronchitis and emphysema. Another example is the lung carbon monoxide diffusing capacity (DLCO) test which determines how effectively gases are exchanged between the blood and the respiratory tract in the lungs. Further, imaging tests, like e.g. chest x-rays or computed tomography (CT) scans, and tests for the protective enzyme, alpha 1-antiprotease (ATT or antitrypsin) which is often deficient in patients having an obstructive lung disease, and bronchoalveolar lavage for determination of inflammatory cells and pro-inflammatory cytokines in the lung may be employed.

The early therapy of an obstructive lung disease in a patient by use of a sodium channel blocker as described above can also be combined with any therapy known in the art for the therapy of an obstructive lung disease. Accordingly, the present invention also relates to the use of at least one sodium channel blocker in the manufacture of a medicament which may also contain further active agents like e.g. anticholinergic agents which relax the bronchial muscles and act as a bronchodilator when inhaled, beta2 agonists being bronchodilators, theophylline, which acts by opening the respiratory tract, improving exchange of gases, reducing shortness of breath, improving mucus clearance, and stimulating the process of breathing, corticosteroids being anti-inflammatory drugs, and osmotically active agents including hypertonic saline or mannitol that improve airway surface hydration by their osmotic action.

The term "patient" as used herein does not underly any specific limitation and includes mammals. In a preferred embodiment of the present invention, the patient is a human.

The present invention further relates to a method of treating a patient having an obstructive lung disease as defined above with at least one sodium channel blocker as defined above, wherein the sodium channel blocker is administered in an early therapy as defined above.

In the following the formulations "βENaC-transgenic" and "*Scnn1b*-transgenic" will be used synonymously.

The figures show:

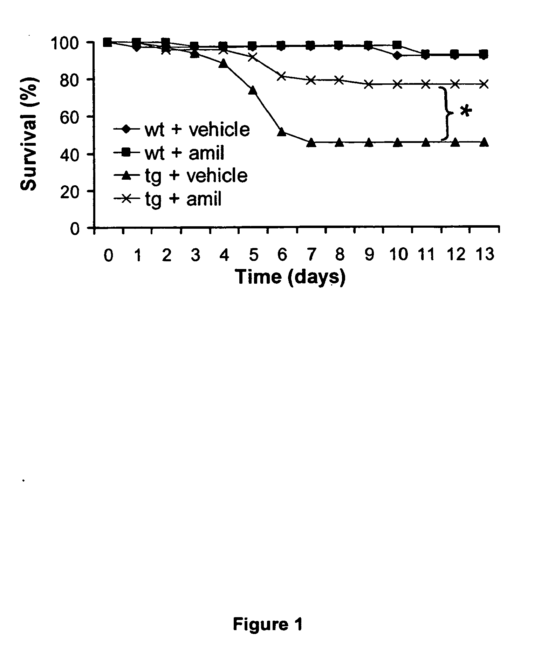

Figure 1 shows that early amiloride treatment (started on the first day of life and continued for 14 days) significantly improved survival of βENaC-transgenic mice compared to vehicle treated βENaC-transgenic littermates. H₂O was used as vehicle in all experiments. Wt, wild-type; tg, βENaC-transgenic. n = 35-48 mice per group. ^{*}*P* = 0.004.

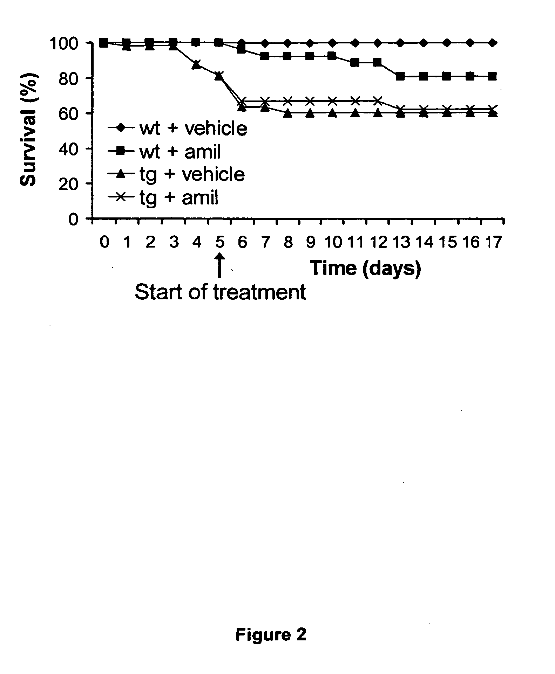

Figure 2 shows that late amiloride treatment (started on postnatal day 5 and continued for 14 days) had no effect on survival of βENaC-transgenic mice compared to vehicle treated βENaC-transgenic littermates. Wt, wild-type; tg, βENaC-transgenic. n = 17-34 mice per group.

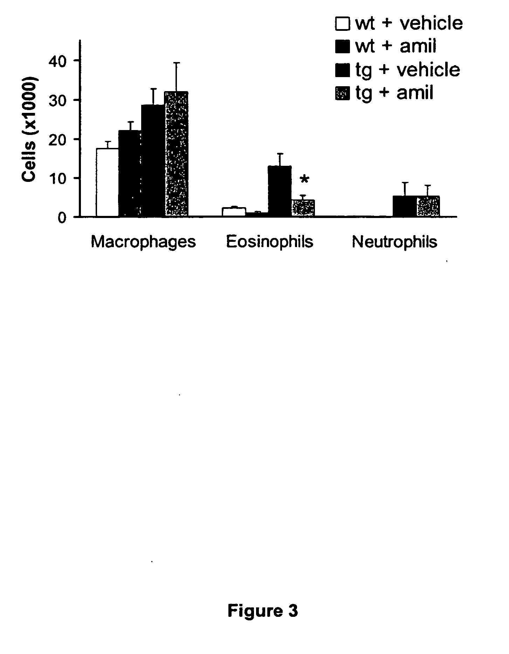

Figure 3 shows that early amiloride treatment (started on first day of life and continued for 14 days) significantly reduced bronchoalveolar lavage (BAL) eosinophil cell counts in βENaC-transgenic mice compared to vehicle treated βENaC transgenic littermates. Means ± SEM, n = 16-34 mice per group. ^{*}*P* = 0.002.

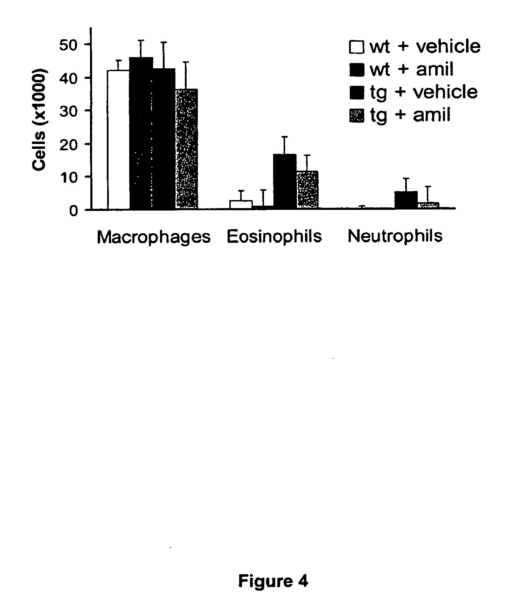

Figure 4 shows that late amiloride treatment (started on postnatal day 5 and continued for 14 days) had no effect on BAL inflammatory cell counts in βENaC transgenic mice compared to vehicle treated βENaC transgenic littermates. Means ± SEM, n = 13-34 mice per group

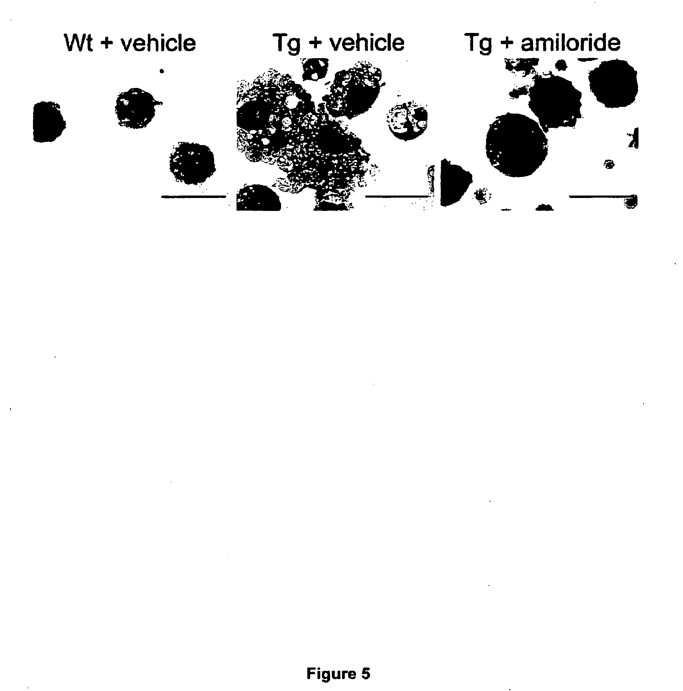

Figure 5 shows that BAL macrophages are activated ('foam cells') in vehicle treated βENaC transgenic mice compared to wild-type littermates. Early amiloride treatment (started on first day of life and continued for 14 days) reduced number of BAL foam cells and average macrophage diameters in βENaC transgenic mice. Giemsa staining. Representative for n = 16-34 mice per group. Scale bars = 20 µm.

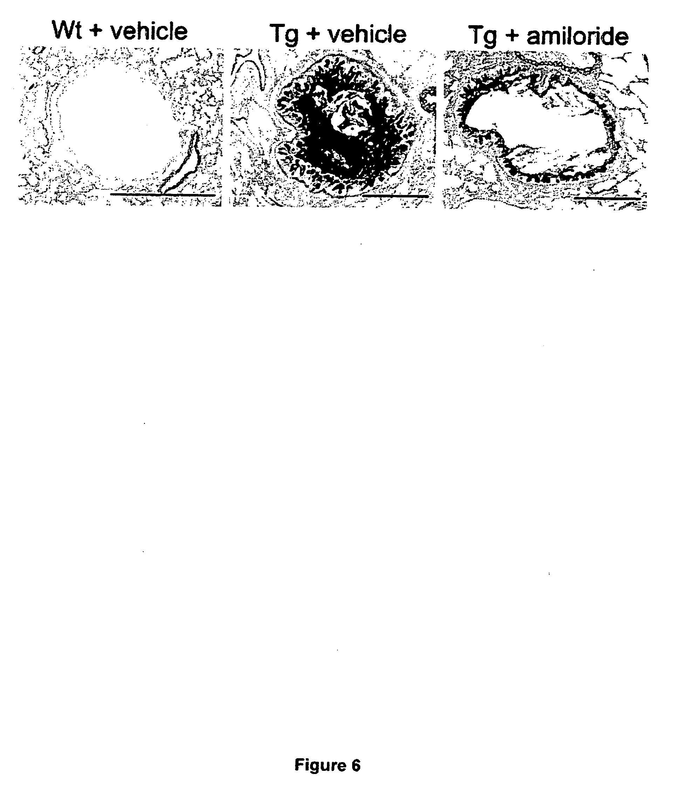

Figure 6 shows that early amiloride treatment (started on first day of life and continued for 14 days) reduced severity of airway mucus plugging in βENaC-transgenic mice (right panel) compared to vehicle (H₂O) treated βENaC-transgenic littermates (middle panel). AB-PAS staining. Scale bars = 500 µm (wt), and 200 µm (tg) respectively. Representative for n = 16-34 mice per group.

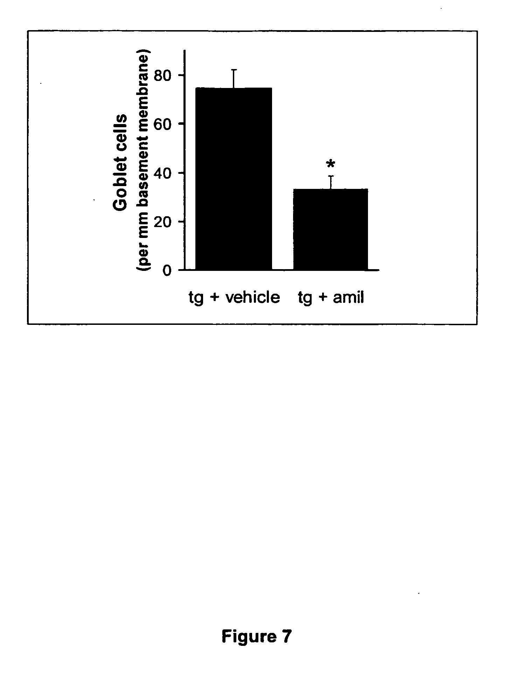

Figure 7 shows that early amiloride treatment (started on first day of life and continued for 14 days) reduced severity of goblet cell metaplasia in βNaC transgenic mice compared to vehicle treated βENaC transgenic littermates. Means ± SEM, n = 14-33 mice per group. ^{*}*P* < 0.001.

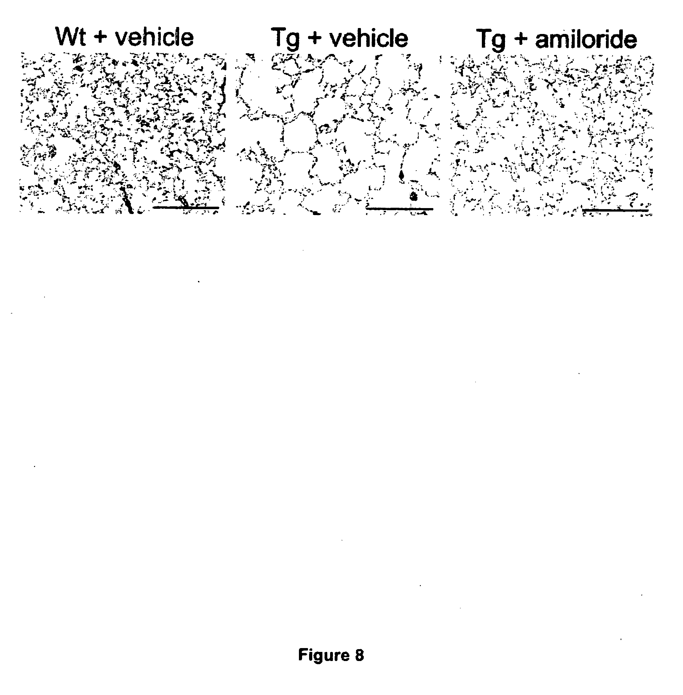

Figure 8 shows that early amiloride treatment (started on first day of life and continued for 14 days) reduced severity of emphysema in βENaC transgenic mice compared to vehicle treated βENaC transgenic littermates. H&E staining. Representative for n = 8-21 mice per group. Scale bars = 200 µm.

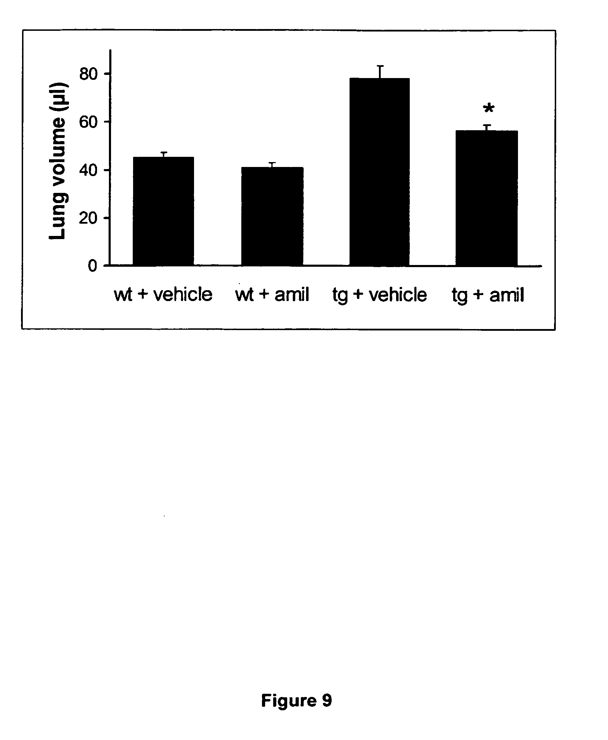

Figure 9 shows that early amiloride treatment (started on first day of life and continued for 14 days) reduced increased lung volume in βENaC transgenic mice compared to vehicle treated βENaC transgenic littermates. Means ± SEM, n = 8-21 mice per group. ^{*}*P* < 0.001.

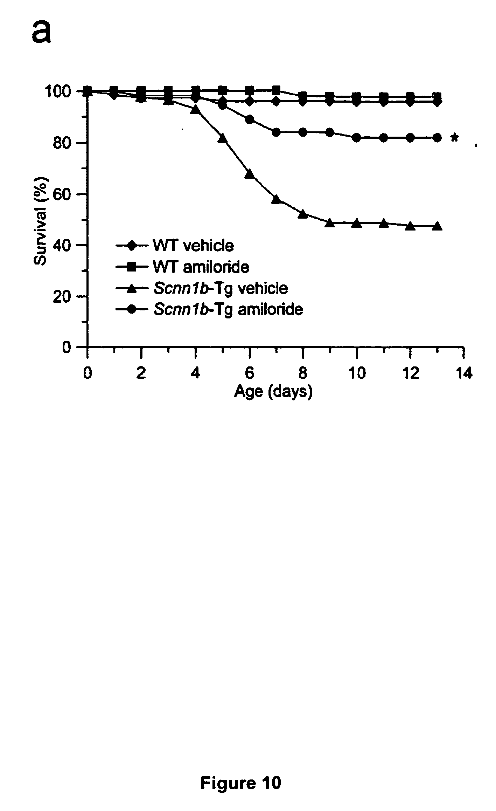

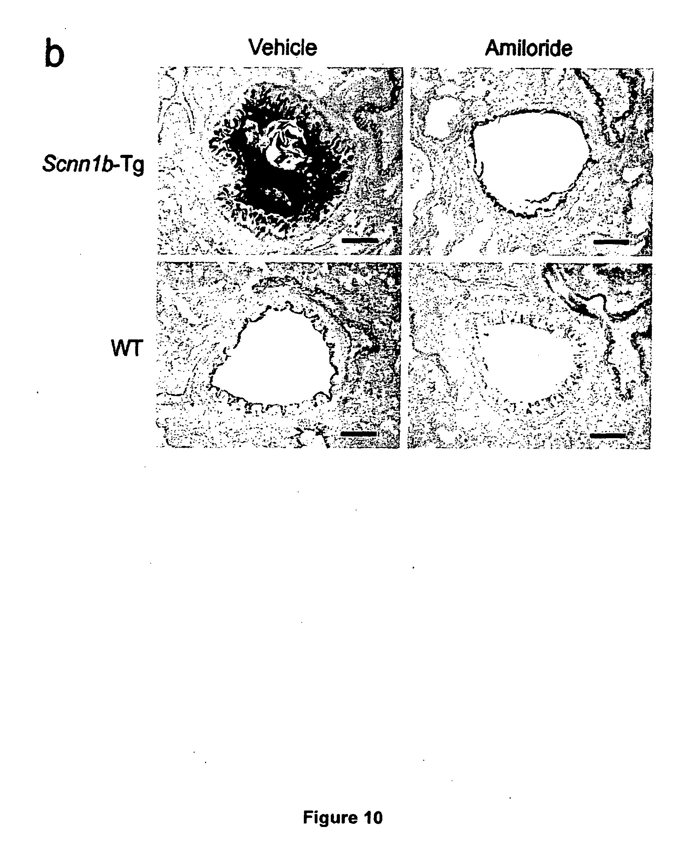

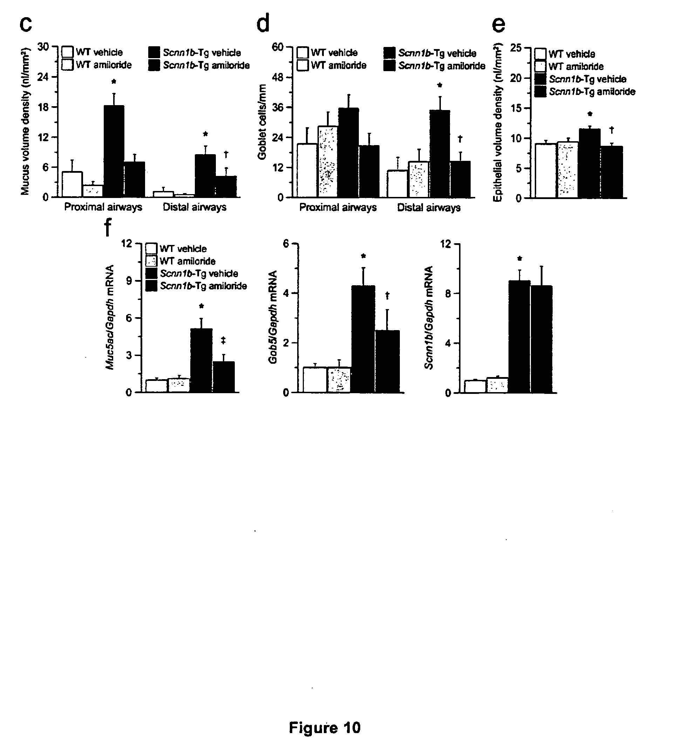

Figure 10 shows that preventive amiloride therapy reduces mortality, airway mucus obstruction and mucus hypersecretion in *Scnn1b*-transgenic mice. (a-e) Effect of preventive amiloride treatment, administered from the first day of life for a period of 2 weeks on survival (a), airway mucus content (b,c), goblet cell counts (d), and epithelial height in *Scnn1b*-transgenic (*Scnn1b*-Tg) mice and wild-type (WT) littermates. (a) Survival curves for *Scnn1b*-transgenic and wild-type mice treated with amiloride or vehicle alone; *n* = 46-86 mice for each group.*, *P* < 0.001 compared with vehicle-treated *Scnn1b*-transgenic mice. (b) Airway histology of *Scnn1b*-transgenic and wild-type mice after preventive treatment with amiloride or vehicle. Sections were stained with AB-PAS to determine the presence of intraluminal mucus and goblet cells. Representative of *n* = 15-27 mice for each group. (c) Mucus content was determined by measuring the volume density of AB-PAS positive material in proximal and distal main axial airways; *n* = 15-27 mice for each group. *, *P* < 0.001 compared with vehicle-treated wild-type. †, *P* < 0.05 compared with vehicle-treated *Scnn1b*-transgenic. ‡ *P* <0.001 compared with vehicle-treated *Scnn1b-*transgenic. (d) Goblet cell densities in proximal and distal main axial airways were determined from the number of AB-PAS positive epithelial cells per mm of the basement membrane; *n* = 15-27 mice for each group. *, *P* < 0.01 compared with vehicle-treated wild-type. †, *P* < 0.05 compared with vehicle-treated *Scnn1b*-transgenic. (e) Epithelial height was determined by measuring the volume density of the epithelium in distal main axial airways; *n* = 15-27 mice for each group. *, *P* < 0.001 compared with vehicle-treated wild-type. †?, *P* < 0.01 compared with vehicle-treated *Scnn1b*-transgenic. (f) Expression levels of *Muc5ac, Gob5* and *Scnn1b* transcripts in lungs from wild-type and *Scnn1b-*transgenic mice after 2 weeks of preventive amiloride treatment. *n* = 13-15 mice for each group. *, *P* = 0.001 compared with vehicle-treated wild-type. †, *P* < 0.05 compared with vehicle-treated *Scnn1b*-transgenic. ‡ *P* < 0.01 compared with vehicle-treated *Scnn1b*-transgenic.

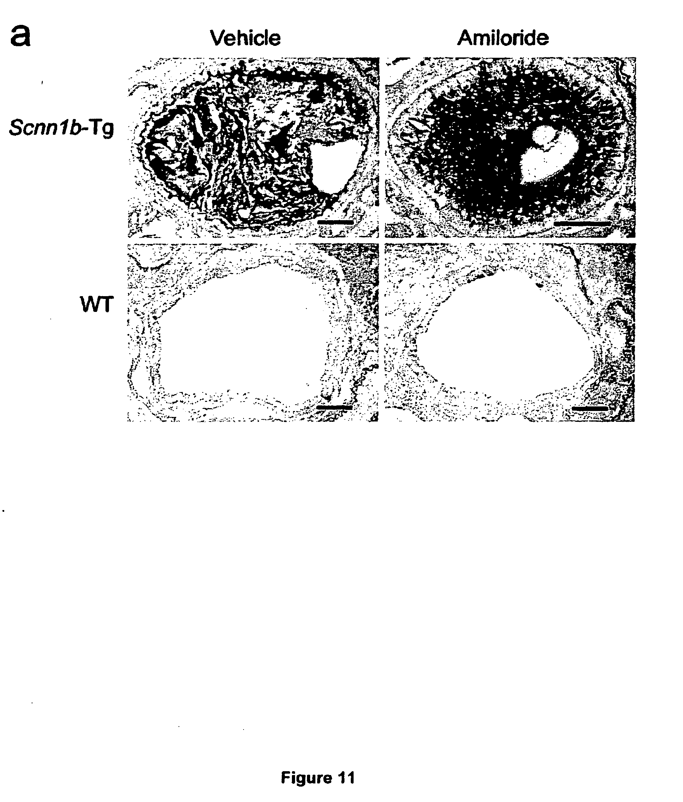

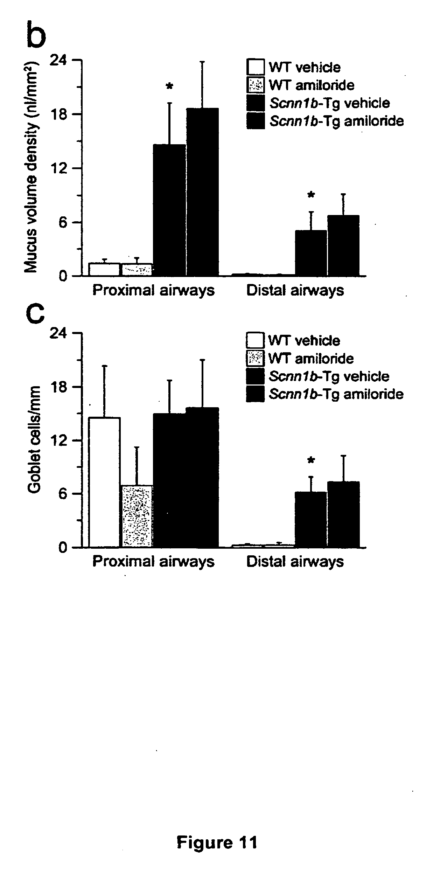

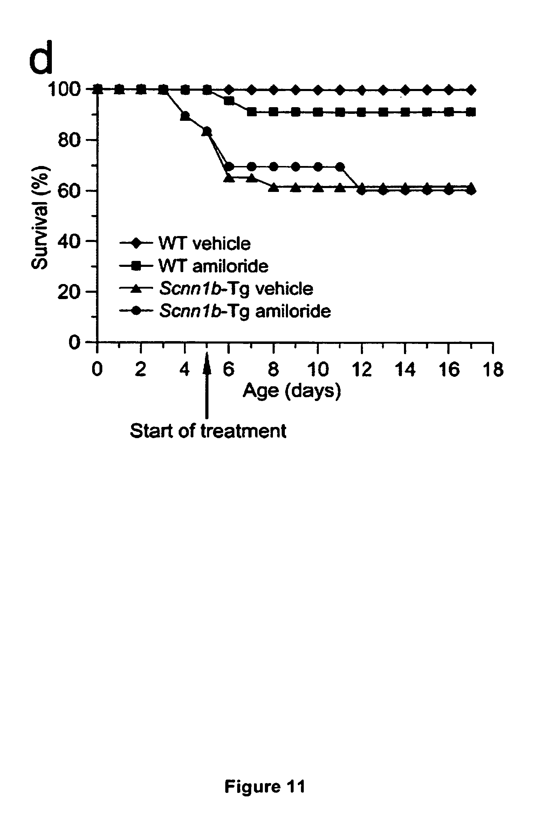

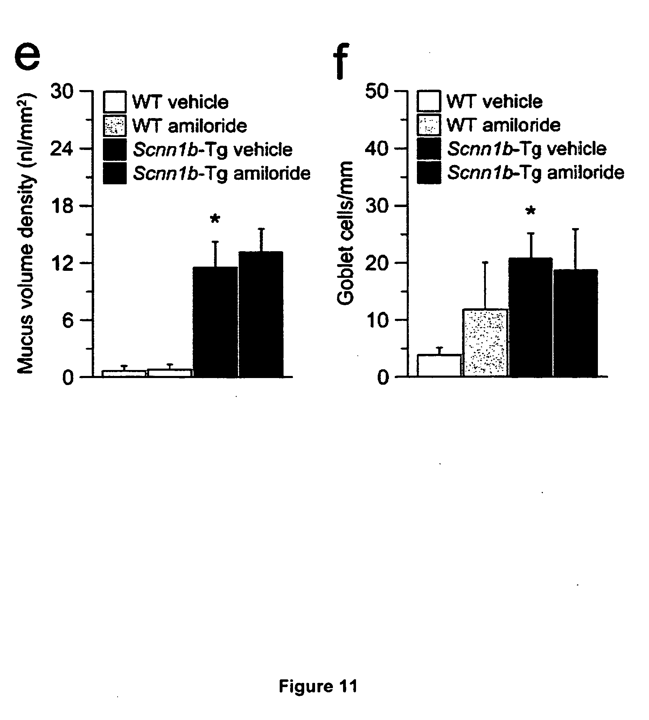

Figure 11 shows that late amiloride treatment in *Scnn1b*-transgenic mice with established chronic obstructive lung disease has no effects on airway mucus obstruction, goblet cell metaplasia and pulmonary mortality. (a-c) Effect of late amiloride treatment, administered from the age of 4 weeks for a period of 2 weeks on airway mucus content (a,b) and goblet cell counts (c) in adult *Scnn1b*-transgenic (*Scnn1b-*Tg) mice and wild-type (WT) littermates; *n* = 9-11 mice for each group. (a) Airway histology from adult *Scnn1b*-transgenic mice and wildtype littermates after administration of amiloride or vehicle for 2 weeks stained with ABPAS to determine the pretence of intraluminal mucus and goblet cells. (b) Mucus content was determined by measuring the volume density of AB-PAS positive material in proximal and distal main axial airways. *, *P* < 0.01 compared with vehicle-treated wild-type. (c) Goblet cell densities in proximal and distal main axial airways were determined from the number of AB-PAS positive epithelial cells per mm of the basement membrane. *, *P* < 0.001 compared with vehicle-treated wild-type. (d-f) Effect of amiloride treatment, administered from the age of 5 days for a period of 2 weeks on survival (d), airway mucus content (e), and goblet cell counts (f) in juvenile *Scnn1b-*transgenic (*Scnn1b*-Tg) mice and wild-type (WT) littermates. (d) Survival curves for *Scnn1b*-transgenic and wild-type mice 21 treated with amiloride or vehicle alone from the age of 5 days; n = 18-34 mice for each group. (e) Mucus content in proximal airways; *n* = 7-11 mice for each group. *, *P* < 0.001 compared with vehicle-treated wild-type. (f) Goblet cell counts in proximal airways; *n* = 7- 11 mice for each group. *, *P* < 0.001 compared with vehicle-treated wild-type.

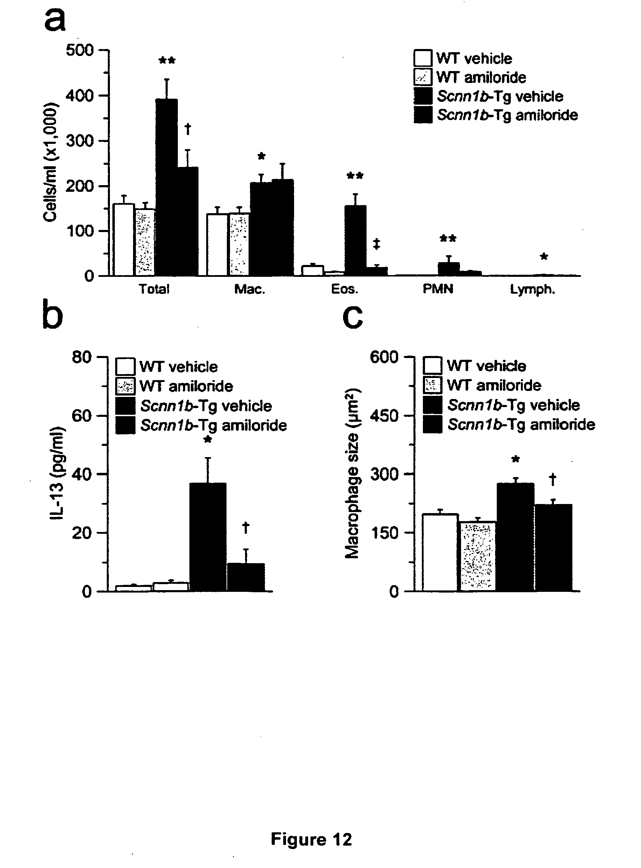

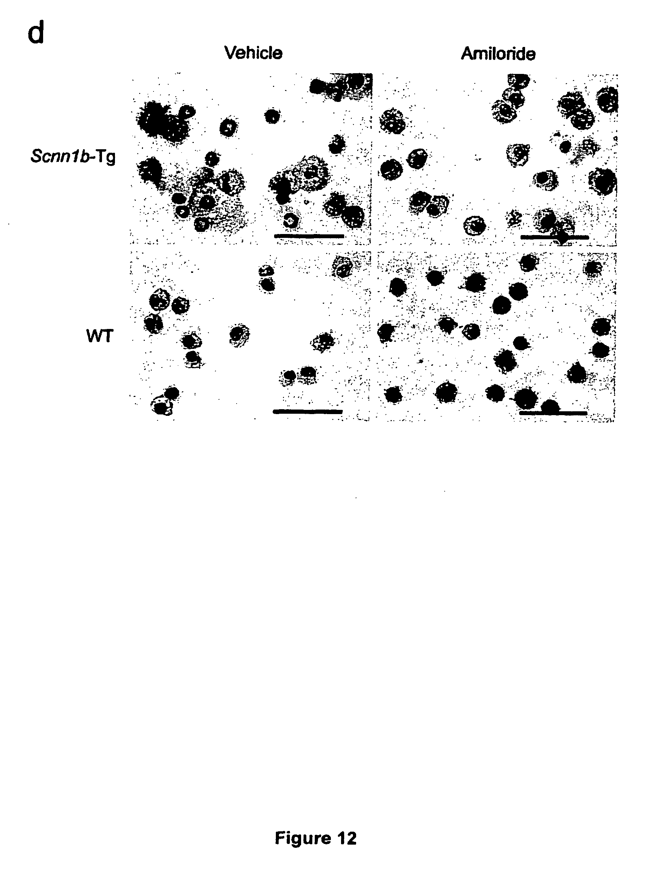

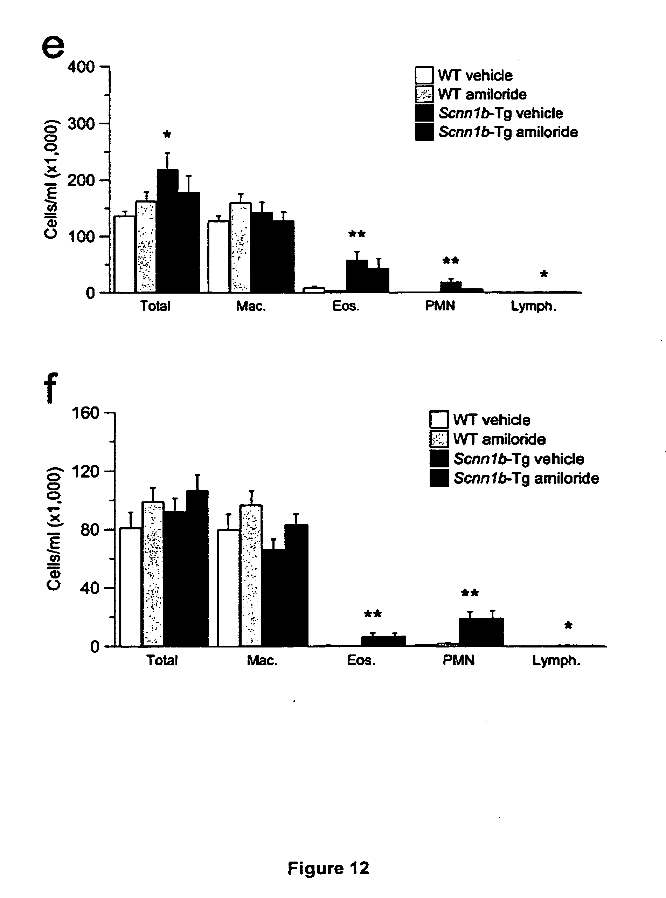

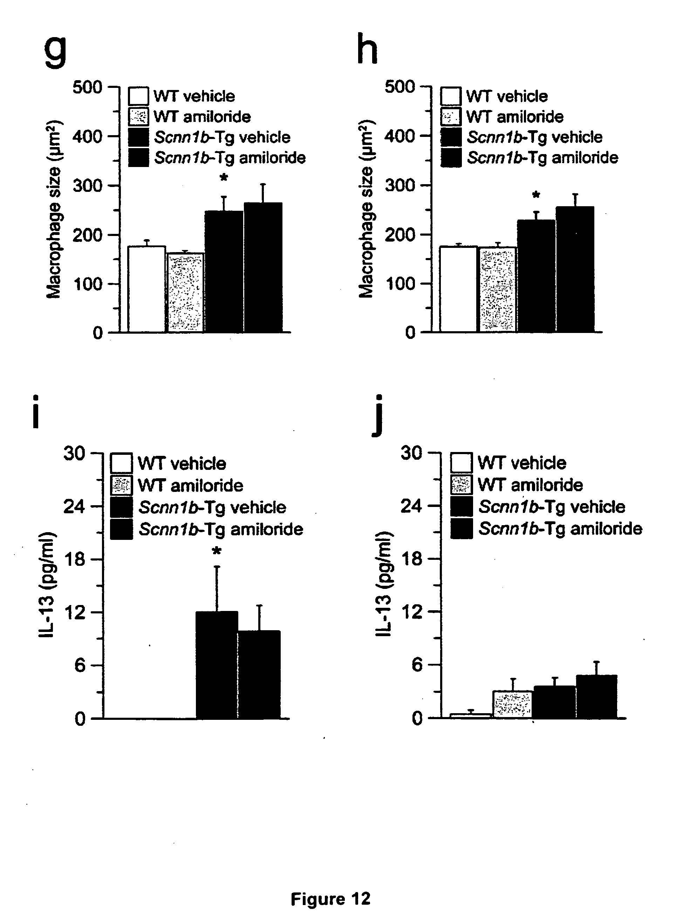

Figure 12 shows that preventive, but not late amiloride therapy reduces airway inflammation in *Scnn1b*-transgenic mice. (a-d) Effect of preventive treatment with amiloride or vehicle alone, administered from the first day of life for a period of 2 weeks, on inflammatory cell counts (a), concentration of the TH2 cytokine IL-13 (b), macrophage size (c) and macrophage morphology (d) in BAL from *Scnn1b*-transgenic (*Scnn1b*-Tg) mice and wildtype (WT) littermates. (a) BAL cell counts; *n* = 27-40 mice for each group. *, *P* < 0.05 compared with vehicle-treated wild-type. ** *P* < 0.001 compared with vehicle-treated wild-type. †?, *P* < 0.05 compared with vehicle-treated *Scnn1b*-transgenic. ‡ *P* < 0.001 compared with vehicle-treated *Scnn1b*-transgenic. (b) IL-13 concentration in BAL ; *n* = 7- 28 mice for each group. *, *P* < 0.01 compared with vehicle-treated wild-type. †?, *P* < 0.01 compared with vehicle-treated *Scnn1b*-transgenic. (c) Size of BAL macrophages ; *n* = 14- 30 mice for each group. *, *P* < 0.001 compared with vehicle-treated wild-type. †?, *P* < 0.01 compared with vehicle-treated *Scnn1b*-transgenic. (d) Morphology of BAL macrophages (stained with May Grünwald Giemsa). Representative of *n* = 27-40 mice for each group. (e-j). Effect of late amiloride treatment, administered from the age of 5 days (e,g,i) or 4 weeks (f,h,j) for a period of 2 weeks, on cell counts (e,f), macrophage size (g,h), and IL-13 concentrations in BAL (i,j) from *Scnn1b*-transgenic and wild-type mice. (e,f) BAL cell 22 counts after treatment with intranasal amiloride or vehicle from the age of 5 days (e) or 4 weeks (f); *n* = 13-34 mice for each group. *, *P* < 0.01 compared with vehicle-treated wildtype. **, *P* < 0.001 copmared with vehicle-treated wild-type. (g,h) Size of BAL macrophages after treatment from 5 days (g) or 4 weeks (h); *n* = 7-11 mice for each group. *, *P* < 0.05 compared with vehicle-treated wild-type. (i,j) IL-13 concentration in BAL after treatment from 5 days (i) or 4 weeks (j); *n* = 4-10 mice for each group. *, *P* < 0.01 compared with vehicle-treated wild-type.

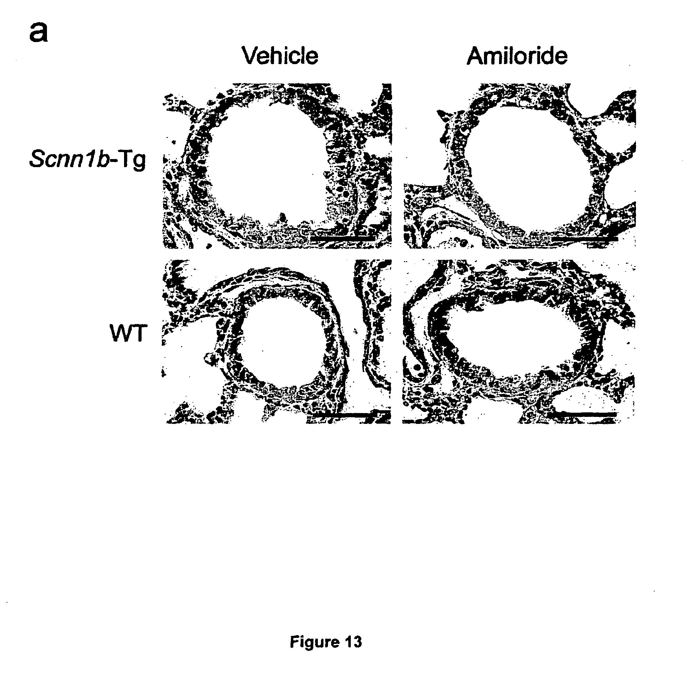

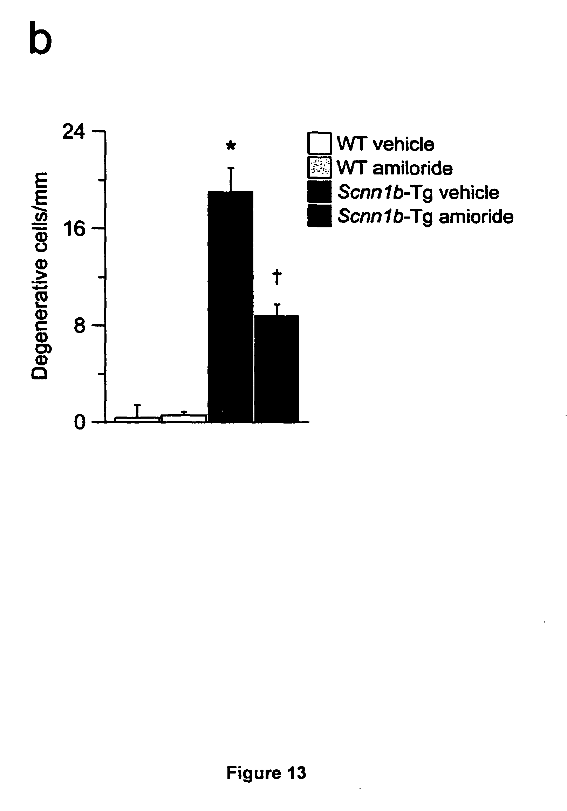

Figure 13 shows that preventive amiloride therapy reduces airway epithelial necrosis in *Scnn1b*-transgenic mice. (a,b) Effect of preventive amiloride treatment, administered from the first day of life for a period of 3 days on airway histology (a), and numbers of degenerative airway epithelial cells (b) in Scnn1b-transgenic (*Scnn1b*-Tg) mice and wild-type (WT) littermates. (a) Airway histology of 3 day old *Scnn1b*-transgenic and wild-type mice after preventive treatment with amiloride or vehicle alone. Sections were stained with H&E to determine the numbers of degenerative airway epithelial cells (arrows). Representative of *n* =7-12 mice for each group. (b) The level of airway epithelial necrosis was determined from the number of degenerative epithelial cells per mm of the basement membrane; *n* = 7- 12 mice for each group. *, *P* = 0.001 compared with vehicle-treated wild-type. †, *P* < 0.001 compared with vehicle-treated *Scnn1b*-transgenic.

The present invention advantageously provides a therapy for the successful treatment of obstructive lung diseases by applying a specific sodium channel blocker like amiloride or a derivative thereof in a living organism as an early therapy. It has surprisingly been found that by the intrapulmonary application of a sodium channel blocker in an early stage of a diagnosed disease, the obstructive lung disease can be cured and disorders associated with said disease like e.g. pulmonal mortality, pulmonal inflammation, mucus obstruction, goblet cell metaplasia, and emphysema can be reduced. Additionally, it has been shown that a preventive sodium channel blocker therapy protects epithelial cells from necrosis and, thus, reduces a strong stimulus for airway inflammation. These superior results are achieved by an early therapy of the patients, i.e. at a stage of the disease when mucus obstruction has not been developed and further secondary changes of the lung, like e.g. goblet cell metaplasia, chronic inflammation of the respiratory tract or emphysema, are not apparent.

The present invention will now be further illustrated in the following examples without being limited thereto.

### Examples

### Example 1

The β-ENaC transgenic mouse has been used as an animal model for chronic obstructive lung diseases of humans to test, whether chronic obstructive lung disease can be treated successfully in a living organism by an early therapy with a sodium channel blocker, i.e. by starting treatment before the development of mucus obstruction and secondary changes of the lung occur. Similar to humans with chronic obstructive lung diseases including CF, CLD, asthma and COPD, the lungs of β-ENaC transgenic mice are normal at birth. Subsequently, β-ENaC transgenic mice develop a spontaneous lung disease that has great similarities to said chronic obstructive lung diseases in humans. At 5 days of age, β-ENaC transgenic mice have already developed significant mucus obstruction and airway inflammation that causes death due to respiratory failure in -50% of β-ENaC transgenic mice in the first 2 weeks of live. For this reason, we treated β-ENaC transgenic mice either from the first day of their lives, i.e. from a date, wherein there were no changes of the lung, or from their fifth day of their lives, i.e. from a date at which mucus obstructions and inflammation of the respiratory tract already existed, with an intrapulmonal application of amiloride.

Intrapulmonal application of amiloride in neonatal mice was achieved by intranasal (i.n.) application of amiloride at a concentration of 3 g/l in a volume of 1 ml/kg body weight, equivalent to a dose of 3 mg/kg body weight. Water was used as vehicle and β-ENaC transgenic mice or wild-type littermate controls were treated three times per day with amiloride or vehicle alone for a period of 2 weeks. To prevent systemic side effects, i.e. possible dehydration due to inhibition of sodium channels in the kidney, in case part of the intranasally applied amiloride was swallowed and absorbed systemically, amiloride-treated mice received concomitant subcutaneous (s.c.) injections with isotonic sodium chloride solution (NaCl 0.9%).

Animals were monitored daily, and deceased mice were genotyped and mortality curves constructed for all treatment groups. At the end of the 2 week treatment cycle, surviving mice were euthanized, and lungs evaluated for several independent clinically relevant outcome measures, including bronchoalveolar lavage to determine therapeutic effects on pulmonary inflammatory cell counts; histopathology, morphometry and lung volume measurements determine effects on mucus obstruction, goblet cell metaplasia and emphysema.

The results of said tests in β-ENaC transgenic mice showed for the first time that by an early therapy of chronic obstructive lung diseases with the sodium channel blocker amiloride significant therapeutic effect can be achieved in a living organism. Accordingly, an intrapulmonal application of amiloride in β-ENaC transgenic mice which have been treated from their first day of life on for a period of 2 weeks resulted in a significant inhibition of the pulmonal mortality (Fig. 1), a significant inhibition of the pulmonal inflammation, as determined from bronchoalveolar lavage studies (Fig. 3, 5), a significant inhibition of the mucus obstruction and goblet cell metaplasia, as determined from histopathology studies (Fig. 6, 7) as well as a significant inhibition of emphysema, as determined from histopathology and lung volume studies, when compared to vehicle treated β-ENaC transgenic mice. A later beginning of the therapy, from the fifth day of the life on, i.e. at a time point when already a progressed lung disease with airway mucus obstruction and inflammation existed in β-ENaC transgenic mice, had no therapeutic effects in the mouse model any more, i.e. mucus plugging induced mortality and pulmonary inflammation were not different in amiloride treated versus vehicle treated β-ENaC transgenic mice (Fig. 2, 4).

### Example 2

### Methods

**Experimental animals**. All animal studies were approved by the Regierungspräsidium Karlsruhe, Germany. The generation of *Scnn1b-*transgenic mice (line 6608) has been previously described (Mall,M., Grubb,B.R., Harkema,J.R., O'Neal,W.K. & Boucher,R.C. Increased airway epithelial Na(+) absorption produces cystic fibrosis-like lung disease in mice. Nat. Med 10, 487-493 (2004)). The colony was maintained on a mixed genetic background (C3H/HeN x C57BL/6N), and *Scnn1b*-transgenic mice were identified by PCR. Wild-type littermates served as controls in all experiments. Mice were housed in a pathogen-free animal facility and had free access to chow and water.

**Amiloride treatment**. Amiloride hydrochloride (Sigma) was dissolved in sterile distilled water (ddH₂O). Newborn, 5-day, and 4-week-old *Scnn1b*-transgenic mice and wild-type littermates were treated by intranasal instillation of amiloride (10 mmol/l; 1µl/g body weight; 3 times per day) or vehicle (ddH₂O) alone for a period of 13-14 days. Pulmonary deposition studies in newborn mice demonstrated that ∼4% of the amiloride dose delivered by intranasal instillation was deposited into the lungs. During amiloride treatment, growth and survival were monitored, and deficits in body mass observed in amiloride-treated mice were replaced by subcutaneous injections of isotonic saline (NaCl 0.9%). 12 hours after the last treatment, BAL was performed, lungs were removed for histology, morphometry and transcript expression studies, and serum and urine were sampled to determine renal effects of absorbed amiloride on Na+ and K₊ concentrations. Endpoint studies were performed by an investigator blinded to the genotype and the treatment of the mice.

**BAL cell counts and cytokine measurements**. Mice were deeply anesthetized via intraperitoneal injection of a combination of ketamin/xylazin (120 mg/kg and 16 mg/kg, respectively), the trachea cannulated, and lungs lavaged with PBS. Samples were centrifuged and the cell-free bronchoalveolar lavage (BAL) fluid was stored at -80°C. Total cell counts were determined and differential cell counts performed on cytospin preparations, as previously described (Mall,M., Grubb,B.R., Harkema,J.R., O'Neal,W.K. & Boucher,R.C. Increased airway epithelial Na(+) absorption produces cystic fibrosis-like lung disease in mice. Nat. Med 10, 487-493 (2004)). Macrophage size was determined by measuring their surface area using Analysis B image analysis software (Olympus). IL- 13 concentrations were measured in BAL using ELISA (R&D Systems) according to manufacturer's instructions.

**Histology and airway morphometry**. Anesthetized mice were killed by exsanguination. Lungs were removed through a median sternotomy, fixed in 4% buffered formalin, and embedded in paraffin. Lungs were sectioned at the level of the proximal intra-pulmonary main axial airway near the hilus, and at the distal intra-pulmonary axial airway, at 1000 µm (2 to 3 week old mice) or 1500 µm (6 week old mice) distal to the hilus. Sections were cut at 5 µm and stained with hematoxylin and eosin (H&E) or alcian blue periodic acid-Schiff (AB-PAS). For quantitative assessment of airway mucus obstruction, we used Analysis B image analysis software (Olympus) to determine mucus volume density. In brief, images of airway sections were taken with an Olympus IX-71 microscope (Olympus) at a magnification of 10x. The length of the airway boundary, as defined by the epithelial basement membrane, was measured by the interactive image measurement tool, and the AB-PAS positive surface area within this boundary was measured by phase analysis according to the automatic threshold settings of the software. The volume density of airway mucus, representing the volume of airway mucus content per surface area of the basement membrane (nl/mm²), was determined from the surface area of AB-PAS positive mucus and the basement membrane length. The volume density of the airway epithelium was determined as a measure of epithelial height. Goblet cells were identified by the presence of intra-cellular AB-PAS positive material, and degenerative airway epithelial cells were identified by morphologic criteria (i.e. cell swelling with cytoplasmic vacuolization). Numeric cell densities were quantitated by counting epithelial cells per mm of the basement membrane. All morphometric measurements were performed by an investigator blinded to the genotype and the treatment of the mice.

**Real-time RT-PCR**. Lungs were stored in RNAlater (Applied Biosystems) and total RNA was isolated using Trizol reagent (Invitrogen). RNA integrity was verified by agarose gel electrophoresis, and cDNA obtained by reverse transcription of 2 µg of total RNA (Superscript III RT; Invitrogen). Real-time PCR for *Muc5ac*, *Gob5*, *Scnn1b* and *Gapdh* was performed on an Applied Biosystems 7500 Real Time PCR System using TaqMan universal PCR master mix and inventored TaqMan gene expression assays according to the manufacturer's instructions (Applied Biosystems). Relative fold changes in target gene expression were calculated from the efficiency of the PCR reaction and the crossing point deviation between samples from the four treatment groups, and determined by normalization to expression of the reference gene *Gapdh,* as previously described.

Statistics. All data were analyzed with SigmaStat version 3.1 (Systat Software) and are reported as mean ± S.E.M. We performed statistical analyses using Student's t-test, Mann- Whitney Rank Sum test, One Way Analysis of Variance (ANOVA), Kruskal-Wallis ANOVA on Ranks and Kaplan-Meier survival analysis as appropriate, and P < 0.05 was accepted to indicate statistical significance.

We used the *Scnn1b*-transgenic mouse as a model of chronic obstructive lung disease (Mall,M., Grubb,B.R., Harkema,J.R., O'Neal,W.K. & Boucher,R.C. Increased airway epithelial Na(+) absorption produces cystic fibrosis-like lung disease in mice. Nat. Med 10, 487-493 (2004); Frizzell,R.A. & Pilewski,J.M. Finally, mice with CF lung disease. Nat. Med 10, 452-454 (2004)) and compared the effects of preventive amiloride treatment versus amiloride intervention after the onset of lung disease on survival, airway mucus obstruction, epithelial necrosis, airway remodeling, and airway inflammation.

The lungs of *Scnn1b*-transgenic mice are structurally normal at birth, but develop central airway mucus obstruction in the first days of life. To evaluate effects of preventive amiloride therapy on chronic obstructive lung disease, amiloride administration to *Scnn1b*-transgenic mice was started on the first day of life, i.e. prior to the onset of lung disease, utilizing a protocol of intranasal administration of amiloride (10 mmol/l; 1µl/g body weight) or vehicle (ddH₂O) alone 3 times daily for a period of 2 weeks. Wild-type littermates were treated with the same protocol to assess for pulmonary toxicity of amiloride therapy. Renal effects of absorbed amiloride were determined by measuring Na+ and K+ concentrations in serum and urine and weight loss due to diuresis. Volume losses were replaced by subcutaneous injections of isotonic saline (NaCl 0.9%).

We first measured the effect of preventive amiloride therapy on survival. Similar to the spontaneous pulmonary mortality observed in previous studies, vehicle-treated *Scnn1b*-transgenic mice exhibited a mortality rate of -50 % (Fig. 10a). Preventive amiloride treatment resulted in a delayed onset with an overall reduction of pulmonary mortality by -70 % in *Scnn1b*-transgenic mice. Amiloride had no adverse effects on survival in wildtype littermates (Fig. 10a).

We measured the effects of preventive amiloride therapy on mucus obstruction, epithelial remodeling with goblet cell metaplasia and epithelial thickening, and mucus hypersecretion in intrapulmonary airways in surviving *Scnn1b*-transgenic mice. Airway mucus content was significantly elevated in vehicle-treated *Scnn1b*-transgenic mice versus wild-type littermates (Fig. 10b,c). Preventive amiloride treatment significantly reduced airway mucus obstruction to near normal values in proximal and distal airway regions of *Scnn1b*-transgenic mice (Fig. 10b,c). Further, early amiloride therapy prevented goblet cell metaplasia and epithelial thickening observed in distal airways of vehicle-treated *Scnn1b-*transgenic mice (Fig. 10d,e). Inhibition of goblet cell metaplasia and airway mucus obstruction was paralleled by a significant reduction of transcript levels of the goblet cell marker *Gob5* and the airway mucin *Muc5ac* in lungs from amiloride-treated compared to vehicle-treated *Scnn1b*-transgenic mice (Fig. 10f). In contrast, preventive ENaC blocker therapy had no effect on expression of *Scnn1b* in lungs from *Scnn1b*-transgenic mice (Fig. 10f), indicating that therapeutic effects of amiloride were conferred by pharmacological inhibition of ENaC-mediated Na+ absorption rather than reduced *Scnn1b* expression.

Evaluation of lungs from vehicle- and amiloride-treated wild-type mice did not reveal any signs of pulmonary toxicity caused by amiloride therapy. Specifically, preventive amiloride therapy did not alter airway mucus content, goblet cell numbers, epithelial height or *Gob5* and *Muc5ac* expression in amiloride-treated compared to vehicle-treated wild-type mice (Fig. 10b-f).

Based on the therapeutic benefits of preventive amiloride therapy, we next tested the effect of amiloride administration on mucus obstruction and mucus hypersecretion in adult *Scnn1b*-trtransgenic mice with established chronic obstructive lung disease. We started treatment at the age of 4 weeks, when *Scnn1b*-transgenic mice exhibit chronic airway mucus obstruction, and remodelling with goblet cell metaplasia and epithelial hypertrophy, and continued treatment of *Scnn1b*-transgenic mice and their wild-type littermates by intranasal instillation of amiloride or vehicle alone for a period of 2 weeks as for the preventive amiloride study (Fig. 10). In contrast to preventive therapy, initiating amiloride treatment in adult mice reduced neither proximal nor distal airways mucus obstruction (Fig. 11 a,b), nor goblet cell metaplasia (Fig. 11 c) in amiloride-treated versus vehicle-treated *Scnn1b*-transgenic mice.

Further, we determined if amiloride therapy was still effective when treatment was started at the age of 5 days, i.e. after the onset of proximal mucus plug formation, but prior to the establishment of chronic lung disease in *Scnn1b-*transgenic mice. In contrast to preventive therapy administered from the first day of life (Fig. 10), initiating amiloride treatment in mice that were alive at the age of 5 days for a period of 2 weeks failed to reduce mortality in amiloride-treated versus vehicle treated *Scnn1b*-transgenic mice (Fig. 11d). Further, initiating amiloride treatment at the age of 5 days had no effect on airway mucus obstruction or goblet cell metaplasia in *Scnn1b*-transgenic mice (Fig. 11e,f).

Collectively, these data demonstrate that preventive amiloride treatment is effective in reducing airway mucus obstruction, airway remodeling, mucin hypersecretion, and pulmonary mortality, but that these therapeutic effects were abrogated when treatment was started after the onset of chronic obstructive lung disease in *Scnn1b*-transgenic mice.

Additionally, we determined whether preventive amiloride therapy had therapeutic effects on airway inflammation in *Scnn1b*-transgenic mice. Consistent with a Th2-biased immune system in the neonatal period, spontaneous airway inflammation in 2 week old *Scnn1b*-transgenic mice is predominated by eosinophils associated with morphologically activated macrophages (i.e. foam cells), elevated numbers of neutrophils, and increased levels of the Th2-signalling molecule IL-13. Evaluation of bronchoalveolar lavage (BAL) fluid for inflammatory cells at the end of the 2 week treatment period revealed that eosinophil numbers were significantly reduced in amiloride-treated versus vehicle-treated *Scnn1b*-transgenic mice (Fig. 12a). This reduction of airway eosinophilia was paralleled by a significant reduction in IL-13 levels in BAL from amiloride-treated versus vehicle-treated *Scnn1b-*transgenic mice (Fig. 12b). Notably, total macrophage numbers were not changed, but macrophage activation was significantly reduced by preventive amiloride therapy in *Scnn1b*-transgenic mice (Fig. 12c,d). In wildtype littermates, preventive amiloride therapy did not have adverse effects on BAL cellularity, IL-13 concentration, or macrophage morphology (Fig. 12a-d).

Taken together, our results show that preventive inhibition of airway Na+ hyperabsorption was efficient in reducing the chronic airway inflammation characteristic of the chronic obstructive lung disease in *Scnn1b*-transgenic mice. Next, we evaluated the effects of amiloride intervention on airway inflammation in 5 day and 4 week old *Scnn1b*-transgenic mice with established chronic obstructive lung disease. In contrast to the anti-inflammatory effects provided by preventive amiloride therapy, starting amiloride treatment after the onset of lung disease had no effect on elevated BAL inflammatory cell counts (Fig. 12e,f), morphological macrophage activation (Fig. 12g,h), or IL-13 levels in BAL (Fig. 12i,j) from amiloride-treated versus vehicle-treated *Scnn1b*-transgenic mice.

We evaluated the effects of preventive amiloride treatment starting on the first day of life on the occurrence of necrotic epithelial cells in airways of 3 day old *Scnn1b*-transgenic neonates (Fig. 13), since cellular necrosis is a potent trigger for inflammation. Notably, compared to vehicle treatment, preventive administration of amiloride significantly reduced the frequency of necrotic airway epithelial cells in neonatal *Scnn1b*-transgenic mice (Fig. 13a,b) demonstrating that preventive sodium channel blocker therapy protected epithelial cells from necrosis and, thus, reduced a strong stimulus for airway inflammation. The mechanistic links between reduced ASL volume and airway inflammation in the *Scnn1b*-transgenic mouse are likely multiple. Neonatal (but not 4 week old) *Scnn1b*-transgenic mice develop airway epithelial hypoxia and epithelial cell necrosis, likely resulting from combined effects of increased epithelial 02 consumption due to Na+ hyperabsorption and decreased 02 delivery due to airway mucus plugging.

Collectively, our results show for the first time that preventive inhibition of accelerated airway Na+ absorption by the sodium channel blocker amiloride is an effective therapy for chronic obstructive lung disease *in vivo.* Preventive amiloride administration exhibited significant therapeutic benefits by reducing spontaneous pulmonary mortality, epithelial necrosis, airway mucus obstruction and inflammation, providing a proof of concept for a novel therapeutic strategy for chronic obstructive lung disease. In contrast to currently available CF therapies that target secondary pathogenetic events, i.e. anti-infective compounds for the treatment of bacterial infections and inhaled DNAse to antagonize increases in sputum viscoelasticity caused by high levels of DNA released from inflammatory cells, preventive inhibition of increased Na+ absorption constitutes the first pharmacological strategy that targets a proximal mechanism involved in the pathogenesis of CF lung disease. Our observation that amiloride therapy became ineffective when treatment was started after the onset of chronic obstructive lung disease in *Scnn1b*-transgenic mice is consistent with previous clinical trials in CF patients with established CF lung disease. The failure of amiloride inhalation therapy in CF patients was mainly attributed to (i) insufficient pulmonary delivery of amiloride due to limited solubility restricting the amount that could be delivered by a nebulizer, (ii) limited potency, and (iii) limited half-life of amiloride on airway surfaces. Our findings showing that amiloride can be delivered to the lung in therapeutically active quantities prior to the onset of lung disease suggest that airway mucus obstruction and/or airway remodeling were contributing factors to the absence of therapeutic benefits in older *Scnn1b*-transgenic mice and CF patients with established lung disease.

It should be noted that amiloride is inexpensive, readily available and has been in clinical use as a diuretic for many years, and that together with a widespread implementation of CF newborn screening programs, and recent improvements in nebulizer technology allowing enhanced aerosol delivery to the neonatal and infant human lung, this facilitates the translation of preventive amiloride therapy according to the present invention for chronic obstructive lung disease from mice to the clinic.

The results of the above Examples show for a first time that a chronic obstructive lung disease can be treated successfully by an early beginning of therapy with an intrapulmonary application of a specific sodium channel blocker like amiloride or a derivative thereof in a living organism. Specifically, early amiloride therapy had significant therapeutic effects on several independent clinically relevant outcomes including pulmonary mortality, airway mucus obstruction and goblet cell metaplasia, pulmonary inflammation, and development of emphysema. It is important to note that the therapeutic use can be exclusively achieved by an early therapy.

Since there has been no effective therapy for the treatment of mucus obstructions, goblet cell metaplasia, chronic pulmonary inflammation, and emphysemas of obstructive lung diseases available, the therapeutic effects which have been obtained using the above new therapeutic strategy in a mouse model represent a significant advantage when compared to therapies for the treatment of obstructive lung diseases already available. The fact that amiloride is already approved for other indications for humans will facilitate the transfer of this new therapeutic strategy to human beings.

## Claims

1. Sodium channel blocker for use in the prevention or early therapy of an obstructive lung disease in a patient.

2. Sodium channel blocker for use according to claim 1, wherein the sodium channel blocker is selected from the group consisting of amiloride, P2Y2 receptor agonists, and protease inhibitors.

3. Sodium channel blocker for use according to claim 2, wherein the sodium channel blocker is amiloride.

4. Sodium channel blocker for use according to any one of claims 1 to 3, wherein the sodium channel blocker is used in an amount ranging from about 0.1 mg/kg body weight to about 10 mg/kg body weight.

5. Sodium channel blocker for use according to any one of claims 1 to 4, wherein the obstructive lung disease is a chronic obstructive lung disease.

6. Sodium channel blocker for use according to any one of claims 1 to 5, wherein the obstructive lung disease is selected from the group consisting of cystic fibrosis (CF), neonatal chronic lung disease (CLD), and asthma bronchiale.

7. Sodium channel blocker for use according to claim 6, wherein the obstructive lung disease is CF.

8. Sodium channel blocker for use according to any one of claims 1 to 7, wherein the patient is a human.

## Patentansprüche

1. Natriumkanalblocker zur Verwendung für die Vorbeugung oder frühen Therapie einer obstruierenden Lungenerkrankung in einem Patienten.

2. Natriumkanalblocker zur Verwendung nach Anspruch 1, wobei der Natriumkanalblocker ausgewählt ist aus der Gruppe, bestehend aus Amilorid, P2Y2-Rezeptoragonisten und Protease-Inhibitoren.

3. Natriumkanalblocker zur Verwendung nach Anspruch 2, wobei der Natriumkanalblocker Amilorid ist.

4. Natriumkanalblocker zur Verwendung nach einem der Ansprüche 1 bis 3, wobei der Natriumkanalblocker in einer Menge im Bereich von etwa 0,1 mg/kg Körpergewicht bis etwa 10 mg/kg Körpergewicht verwendet wird.

5. Natriumkanalblocker zur Verwendung nach einem der Ansprüche 1 bis 4, wobei die obstruierende Lungenerkrankung eine chronische obstruierende Lungenerkrankung ist.

6. Natriumkanalblocker zur Verwendung nach einem der Ansprüche 1 bis 5, wobei die obstruierende Lungenerkrankung ausgewählt ist aus der Gruppe bestehend aus cystischer Fibrose (CF), neonataler chronischer Lungenerkrankung (CLD) und Asthma bronchiale.

7. Natriumkanalblocker zur Verwendung nach Anspruch 6, wobei die obstruierende Lungenerkrankung CF ist.

8. Natriumkanalblocker zur Verwendung nach einem der Ansprüche 1 bis 7, wobei der Patient ein Mensch ist.

## Revendications

1. Inhibiteur de canal sodique à utiliser dans la prévention ou le traitement précoce d'une maladie pulmonaire obstructive sur un patient.

2. Inhibiteur de canal sodique à utiliser selon la revendication 1, dans lequel l'inhibiteur de canal sodique est choisi dans le groupe constitué d'amiloride, d'agonistes de récepteur P2Y2 et d'inhibiteurs de protéase.

3. Inhibiteur de canal sodique à utiliser selon la revendication 2, dans lequel l'inhibiteur de canal sodique est l'amiloride.

4. Inhibiteur de canal sodique à utiliser selon l'une quelconque des revendications 1 à 3, dans lequel l'inhibiteur de canal sodique est utilisé dans une quantité comprise entre environ 0,1 mg/kg de masse corporelle et environ 10 mg/kg de masse corporelle.

5. Inhibiteur de canal sodique à utiliser selon l'une quelconque des revendications 1 à 4, dans lequel la maladie pulmonaire obstructive est une maladie pulmonaire obstructive chronique.

6. Inhibiteur de canal sodique à utiliser selon l'une quelconque des revendications 1 à 5, dans lequel la maladie pulmonaire obstructive est choisie dans le groupe constitué de la fibrose kystique (CF), la maladie pulmonaire chronique néonatale (CLD) et l'asthme bronchique.

7. Inhibiteur de canal sodique à utiliser selon la revendication 6, dans lequel la maladie pulmonaire obstructive est la CF.

8. Inhibiteur de canal sodique à utiliser selon l'une quelconque des revendications 1 à 7, dans lequel le patient est un être humain.
